# EUROPÄISCHE PATENTSCHRIFT

(11) **EP 3 858 302 B1**
(45) Veröffentlichungstag und Bekanntmachung des Hinweises auf die Patenterteilung: **28.06.2023**
(21) Anmeldenummer: 20175115.3
(22) Anmeldetag: 17.05.2020
(51) Int. Cl.: A61F 5/01

(54) **GELENK FÜR EINE ORTHOPÄDISCHE QUENGELSCHIENE**
JOINT FOR AN ORTHOPAEDIC QUENGEL ORTHOTIC
ARTICULATION POUR UN RAIL ÉLASTIQUE ORTHOPÉDIQUE

(30) Priorität: 29.01.2020 DE 202020100487 U
(43) Veröffentlichungstag der Anmeldung: 04.08.2021
(73) Patentinhaber: Heinrich Caroli GmbH, 77933 Lahr (DE)
(72) Erfinder: Niedermaier, Simon, 77933 Lahr/Schwarzwald (DE)
(74) Vertreter: RGTH

(56) Entgegenhaltungen:
- DE-A1-102015 012 321
- TW-A- 200 407 216
- TW-A- 201 107 084
- US-A1- 2004 251 105

## Beschreibung

Die vorliegende Erfindung betrifft ein Gelenk für eine orthopädische Quengelschiene sowie eine orthopädische Quengelschiene, wie in den Ansprüchen definiert.

### Stand der Technik

Quengelschienen sind aus dem Stand der Technik bekannt. Sie dienen zur Redression beziehungsweise Therapie von Gelenkkontrakturen im Rahmen einer orthetischen Hilfsmittelversorgung. Die Redression ist ein konservatives Therapieverfahren, das Kontrakturen oder Fehlstellungen schrittweise mit manuellen oder apparativen Methoden korrigiert. Diese apparative Behandlung wird durch eine Orthesenschiene durchgeführt, die in Orthesen eingebaut wird. Beispielsweise ist in der EP 2 179 712 A1 eine Orthese zum gestreckten Halten beziehungsweise Auseinanderziehen flexibler knöcherner Strukturen des Körpers, die zur Redression eingesetzt wird, offenbart.

DE 10 2015 012 321 A1 offenbart eine dynamische Gelenkstütze.

US 2004/0251105 A1 betrifft ein Richtungsschalter für ein Gelenk eines Fahrzeuges.

TW 201 107 084 A offenbart einen Ratschenschlüssel, während TW 200 407 216 A eine Schlüsselkeilvorrichtung beschreibt.

### Darstellung der Erfindung: Aufgabe, Lösung, Vorteile

Die Erfindung hat die Aufgabe, ein Gelenk für eine orthopädische Quengelschiene derart weiterzubilden, dass dieses einen einfachen, werkzeuglosen Wechsel zwischen verschiedenen Funktionszuständen erlaubt. Der Gegenstand der Erfindung ist in den Ansprüchen definiert.

Diese Aufgabe wird gelöst durch ein Gelenk für eine orthopädische Quengelschiene, das ein Gelenkkopf mit einem Zahnradabschnitt, einen Gelenkkörper und mindestens ein Sperrelement mit mindestens einem Zahnradabschnitt aufweist.

Dabei weist der Gelenkkörper einen Aufnahmebereich auf, wobei der Gelenkkopf drehbar im Aufnahmebereich angeordnet ist. Ferner ist das mindestens eine Sperrelement schwenkbar im Aufnahmebereich angeordnet.

Der Gelenkkopf und/oder das Sperrelement können über ihre Außenfläche bzw. Mantelfläche im Gelenkkörper geführt und/oder gelagert sein. Ferner können der Gelenkkopf und/oder das Sperrelement über einen Achse geführt und/oder gelagert sein. Diese Achse ist dann vorzugsweise im Zentrum des jeweiligen Bauteiles angeordnet.

Das Sperrelement oder ein Umschaltelement des Gelenks ist in mindestens eine erste Position und eine zweite Position bringbar, wobei in der ersten Position eine Drehbewegung des Gelenkkopfes im Aufnahmebereich in eine erste Richtung freigegeben ist und eine Drehbewegung in die entgegengesetzte zweite Richtung durch ein Eingreifen eines Zahnradabschnittes eines Sperrelementes und des Zahnradabschnittes des Gelenkkopfes gesperrt ist. Insbesondere ist das Umschaltelement schwenkbar im Aufnahmebereich angeordnet.

In anderen Worten ist in der ersten Position eine relative Bewegung zwischen Gelenkkopf und Gelenkkörper durch Rotation des Gelenkkopfes möglich, während dies in die zweite Richtung mittels eines Sperrelementes blockiert ist. Ferner ist in der zweiten Position eine Drehbewegung des Gelenkkopfes im Aufnahmebereich in die zweite Richtung freigegeben und eine Drehbewegung in die entgegengesetzte erste Richtung durch ein Eingreifen eines Zahnradabschnittes eines Sperrelementes und des Zahnradabschnitts des Gelenkkopfes gesperrt. Erneut wird die Drehbewegung in einer Richtung freigegeben, während sie in entgegengesetzte Richtung blockiert ist.

Der Aufnahmebereich des Gelenkkörpers weist in Draufsicht und Längsrichtung insbesondere die Form einer Acht auf. Er kann in einen ersten Bereich und einen zweiten Bereich untergliedert werden. Der erste Bereich und der zweite Bereich sind jeweils zylinderförmig ausgebildet, wobei diese sich derart überlappen, dass in Draufsicht die Form einer Acht des Aufnahmebereichs entsteht. Im ersten Bereich ist insbesondere der Gelenkkopf angeordnet, während im zweiten Bereich insbesondere das mindestens eine Sperrelement und/oder das Umschaltelement angeordnet sind.

Ferner bevorzugt weist der Aufnahmebereich eine erste Durchbrechung, insbesondere im ersten Bereich, auf. Ferner weist der Aufnahmebereich eine zweite Durchbrechung, insbesondere im zweiten Bereich auf. Die zweite Durchbrechung dient insbesondere zum teilweisen Durchführen des Umschaltelementes.

Ferner kann der Gelenkkörper eine Innenwand aufweisen, die den Aufnahmebereich definiert. Zudem kann der Gelenkkörper Befestigungsbohrungen umfassen, die dazu dienen, den Gelenkkörper an einem Schienenelement einer Quengelschiene zu befestigen. Ferner kann der Gelenkkörper Ausnehmungen zur Aufnahme des mindestens einen Sperrelementes umfassen. Ferner kann der Gelenkkörper mindestens eine Ausnehmung zur Aufnahme des mindestens einen Sperrelementes umfassen. Des Weiteren kann der Gelenkkörper zapfenförmige Erhebungen im Bereich dessen Wandung aufweisen, welche in mindestens eine Aussparung eines Schienenelementes eingreifen und den Gelenkkörper gegen eine Verdrehung sichern können.

Bevorzugterweise ermöglicht das mindestens eine Sperrelement benachbarte Sperrzustände mit einem Winkelabstand von 5°. In anderen Worten ermöglicht das mindestens eine Sperrelement verschiedene Sperrzustände, das heißt eine Winkeleinstellung zwischen Gelenkkopf und Gelenkkörper, in die die Drehbewegung in eine Richtung blockiert ist. Sobald eine Drehung in die andere, freigegebene Bewegung ausgelöst wird, verschiebt sich dieser Sperrzustand in diese freigegebene Richtung, da nach der Bewegung in die frei gegebene Richtung, die entgegengesetzt Richtung erneut blockiert ist. Dabei weisen benachbarte Sperrzustände nur einen Winkelabstand von 5° auf, sodass das Gelenk optimal anpassbar ist. Dadurch lässt sich auch die Winkelgradverstellung zur Redression verändern, und zwar ebenfalls werkzeuglos.

Der Gelenkkopf ist vorzugsweise zylinderförmig ausgebildet, wobei sich der Zahnradabschnitt um die Mantelfläche erstreckt. In anderen Worten handelt es sich bei dem Zahnradabschnitt um eine Außenverzahnung. Dabei weist der Zahnradabschnitt mindestens 50 Zähne, vorzugsweise mindestens 60 Zähne, am meisten bevorzugt mindestens 70 Zähne, auf. Durch diese feine Verzahnung wird der nahe Abstand benachbarter Sperrzustände ermöglicht, ferner wird eine stufenweise Drehbewegung mit einer sehr geringen Winkelabstufung ermöglicht.

Ferner weist der Gelenkkopf vorzugweise einen Befestigungsabschnitt auf, der stirnseitig hervorsteht. Dieser Befestigungsabschnitt kann in Form eines Vierkants ausgebildet sein. Er dient zur Befestigung mit einem Schienenelement einer Quengelschiene. Der Gelenkkopf ist insbesondere derart in den ersten Bereich des Aufnahmebereichs eingebracht wird, dass der Befestigungsabschnitt aus dem Aufnahmebereich hervorragt.

Insbesondere dient das Gelenk zum Verbinden zweier Schienenelemente einer Quengelschiene. Die Schienenelemente sind insbesondere stabförmig ausgebildet und mittels des Gelenkes miteinander verbunden, wobei das Gelenk, wie oben beschrieben, unter bestimmten Voraussetzungen, eine relative Bewegung der Schienenelemente zueinander zulässt.

Die Quengelschiene dient dabei zum Quengeln von bewegungseingeschränkten Körpergelenken. Darunter ist eine Behandlung beziehungsweise Therapie von Kontrakturen von Körpergelenken zu verstehen. Als Kontraktur wird eine Funktions- und/oder Bewegungseinschränkung von Körpergelenken bezeichnet.

Eine Kontraktur kann durch die Verkürzung umliegender Weichteile wie Muskeln, Sehnen, Bändern und/oder Faszien entstehen, sodass sich die betroffenen Köpergelenke sowohl aktiv wie auch passiv nicht oder nur schwer und in geringem Maße bewegen lassen. Ferner kann die Bewegung sehr schmerzhaft sein. Die Therapie bzw. Behandlung, deren Ziel eine Verbesserung der Gelenkbeweglichkeit ist, wird auch als Redression bezeichnet.

Dadurch dass das Sperrelement oder ein Umschaltelement in verschiedene Positionen bringbar ist, in denen unterschiedliche relative Bewegungen des Gelenkkopfes zum Gelenkkörper möglich sind, ist ein Richtungswechsel der Drehbewegung des Gelenkes problemlos möglich. Dies kann durch den Einsatz eines Umschaltelementes in Verbindung mit mindestens einem Sperrelement, in anderen Worten durch Betätigen des Umschaltelementes, ermöglicht werden. Dabei können vor allem zwei Sperrelemente, nämlich ein erstes und ein zweites Sperrelement, eingesetzt werden, die jeweils einen Zahnradabschnitt, insbesondere genau einen Zahnradabschnitt, aufweisen. Das erste Sperrelement ist vorzugsweise dazu ausgebildet eine Drehbewegung in der zweiten Richtung in der ersten Position des Umschaltelementes zu sperren, wobei das zweite Sperrelement eine Drehbewegung in die erste Richtung in zweiter Position des Umschaltelementes sperrt. Alternativ kann das Sperrelement in mindestens eine erste Position und eine zweite Position gebracht werden. Dann werden vorzugsweise ein einziges Sperrelement mit mindestens zwei Zahnradabschnitten und insbesondere kein Umschaltelement eingesetzt. Unterschiedliche Zahnradabschnitte des Sperrelementes sorgen in der ersten Position und in der zweiten Position für eine Sperrung der Drehbewegung in jeweils eine Richtung.

Unter dem Begriff "Sperren" ist insbesondere zu verstehen, dass eine Drehbewegung in die besagte Richtung nicht möglich ist. In anderen Worten blockiert das Sperrelement eine entsprechende Drehbewegung.

Insbesondere ist das Sperrelement oder das Umschaltelement in eine dritte Position bringbar, wobei in der dritten Position eine Drehbewegung des Gelenkkopfes im Aufnahmebereich des Gelenkkörpers sowohl in erster Richtung als auch zweiter Richtung freigegeben ist. In anderen Worten handelt es sich dabei um eine Leerlauffunktion beziehungsweise Freistellfunktion, in der eine Drehbewegung in beide Richtungen möglich ist. Die erste Position und die dritte sowie die zweite und die dritte Position unterscheiden sich darin, dass das Sperrelement oder das Umschaltelement um jeweils einen Winkel zwischen 10° und 60°, insbesondere zwischen 20° und 50°, am meisten bevorzugt um einen Winkel von etwa 45°, gedreht ist. Die dritte Position befindet sich insbesondere zwischen der ersten und der zweiten Position.

Die Freistellfunktion ermöglicht unterschiedliche Nutzungsmöglichkeiten, beispielsweise das Gehen, zum Beispiel im innerhäuslichen Bereich, das Ausführen von physiologischen Bewegungen während der Anwendung, die Bewerkstelligung elementarer Grundbedürfnisse, beispielsweise den Toilettengang oder die Nahrungsaufnahme. Ferner ist sie vorteilhaft zu Therapiezwecken, beispielsweise zur Mobilisation oder für Steh- und Gehübungen. In anderen Worten ermöglicht die vorliegende Erfindung eine erweiterte Anwendung und Nutzung im Alltag für den Patienten.

Insgesamt ermöglicht die vorliegende Erfindung somit drei verschiedene Funktionszustände, und zwar in einer ersten Position eine Bewegung in erster Richtung, wobei die Bewegung in zweiter Richtung gesperrt ist, in einer zweiten Position eine Bewegung in zweiter Richtung, wobei die Bewegung in erster Position gesperrt ist, und eine Freistellfunktion, in der eine uneingeschränkte Drehbewegung möglich ist.

Beim Einsatz bei einem Körpergelenk kann somit die erste oder die zweite Position eine Flexion, in anderen Worten Beugung, zulassen, während die andere Position eine Extension beziehungsweise Streckung zulässt. Somit ist eine Redression in Flexion und Extension möglich. Die Redression erfolgt über Druck und/oder Zugkräfte durch eine manuelle Betätigung des Gelenks oder der Quengelschiene bzw. der betroffenen Extremität.

Ferner weist das Gelenk einen Arbeitsbereich auf, der 360° umfasst. Es ermöglicht somit einen maximalen Arbeitsbereich. Insgesamt ist somit das Gelenk bzw. die Quengelschiene universell einsetzbar, sowohl für die untere als auch die obere Extremität, für Kinder, Jugendliche und Erwachsene und für die linke und rechte Körperseite, wobei keine bestimmte Einbaurichtung erforderlich ist, da die verschiedenen Funktionszustände zusammen jegliche Bewegung ermöglichen.

Ferner ist die Umschaltung der verschiedenen Funktionsstände werkzeuglos möglich. Insbesondere wird das Sperrelement oder das Umschaltelement manuell bedient um es in die verschiedenen Positionen zu bringen. Es wird kein Werkzeug, bzw. ein Verstellschlüssel, zum Umschalten verwendet, sodass das vorliegende Gelenk werkzeuglos in die verschiedenen Funktionszustände gebracht werden kann.

Es kann eine Redression durch Eigenkraft ermöglicht werden, in anderen Worten ist unter Eigenkraft die Muskelkraft gemeint, sodass die Redression nicht mittels eines Werkzeuges, beispielsweise über ein Schneckentrieb oder ein Getriebe, forciert wird. Ferner ist die Umschaltung leicht bedienbar. Auch eine Umschaltung der Funktionszustände unter beziehungsweise durch die Kleidung ist ermöglicht. All dies ist von großem Vorteil, beispielsweise bei Patienten, die keine direkte Rückmeldung geben können. Dies sind beispielsweise Kinder oder Wachkomapatienten. In einem solchen Falle hat der "Bediener" ein besseres Gefühl, wie viel Kraft er entgegen der Kontraktur aufbringt.

Insbesondere weist das Gelenk ein erstes Sperrelement und ein zweites Sperrelement auf, wobei das erste Sperrelement und das zweite Sperrelement jeweils einen Zahnradabschnitt aufweisen. In der ersten Position des Umschaltelementes ist eine Drehbewegung des Gelenkkopfes im Aufnahmebereich des Gelenkkörpers in eine erste Richtung freigegeben und eine Drehbewegung in die entgegengesetzte zweite Richtung durch ein Eingreifen des Zahnradabschnittes des ersten Sperrelementes und des Zahnradabschnittes des Gelenkkopfes blockiert, wobei in der zweiten Position des Umschaltelementes eine Drehbewegung des Gelenkkopfes im Aufnahmebereich des Gelenkkörpers in eine zweite Richtung freigegeben und eine Drehbewegung in die entgegengesetzt erste Richtung durch ein Eingreifen des Zahnradabschnittes des zweiten Sperrelementes und des Zahnradabschnittes des Gelenkkopfes gesperrt ist.

Insbesondere weist das mindestens eine Sperrelement einen Befestigungsabschnitt auf, mittels dessen es im Aufnahmebereich des Gelenkkörpers befestigt ist.

Vorzugsweise weist das Sperrelement einen Verbindungsabschnitt auf, der den Zahnradabschnitt und den Befestigungsabschnitt miteinander verbindet. Insbesondere in dem Fall, dass das Gelenk zwei Sperrelemente umfasst, sind diese stäbchenförmig ausgebildet, wobei deren Breite in Richtung des Befestigungsabschnittes zunimmt. Der Verbindungsbereich weist insbesondere eine Innenseite auf und eine Außenseite, wobei die Außenseite in Richtung der Innenwand des Aufnahmebereichs zeigt. Das Sperrelement wird insbesondere mit seinem Befestigungsabschnitt in einer Ausnehmung des Aufnahmebereichs des Gelenkkörpers befestigt.

Insbesondere ist das mindestens eine Sperrelement durch mindestens ein Vorspannelement vorgespannt. Dies dient dazu, dass das Sperrelement fest gegen den Zahnradabschnitt des Gelenkkopfes anstößt und diesen in Eingriff nimmt. Das Vorspannelement ist insbesondere als Druckfeder ausgebildet. Die Druckfeder hat insbesondere ein erstes Ende, das an der Innenwand des Aufnahmebereichs des Gelenkkörpers angeordnet ist und einen zweiten Endbereich, der gegen das mindestens eine Sperrelement drückt, und zwar insbesondere gegen die Außenseite des Verbindungsabschnittes.

Das Sperrelement ist vor allem derart im Aufnahmebereich angeordnet, dass dessen Längsrichtung schräg zur radialen Richtung des Gelenkkopfes steht. Insbesondere steht dessen Längsrichtung etwa in einem Winkel zwischen 5° und 45°, insbesondere in einem Winkel von 10° bis 40°, am meisten bevorzugt in einem Winkel von 15° und 30°, zu einer radialen Richtung des Gelenkkopfes. Dadurch, dass das Vorspannelement gegen die Außenseite des Sperrelementes stößt, wird dies gegen den Zahnradabschnitt des Gelenkkopfes gedrückt. Durch die Schrägstellung wird bei Eingriff des Zahnradabschnitts des Gelenkkopfes und des Zahnradabschnittes des Sperrelementes eine Drehbewegung entgegen der Schrägstellung gesperrt. Eine Drehbewegung entgegen der Schrägstellung bedeutet, dass die Drehbewegung des Gelenkkopfes durch den Eingriff der Zahnradabschnitte das Sperrelement aufstellen würde. In anderen Worten würde die Bewegung das Sperrelement in eine Anordnung bringen wollen die radial zum Gelenkkopf steht.

Insbesondere sind das erste Sperrelement und das zweite Sperrelement jeweils durch ein Vorspannelement, insbesondere eine Druckfeder, vorgespannt.

Bevorzugterweise weist das Umschaltelement einen Druckabschnitt auf, der zwischen dem ersten Sperrelement und dem zweiten Sperrelement angeordnet ist. Insbesondere weist das Umschaltelement ferner einen Umschalthebel auf, wobei das Umschaltelement derart durch die zweite Durchbrechung des Gelenkkörpers angeordnet ist, dass sich der Druckabschnitt innerhalb des Aufnahmebereichs des Gelenkkörpers befindet, während der Umschalthebel auf der entgegengesetzten Seite des Gelenkkörpers angeordnet ist. Eine Betätigung des Umschalthebels wird durch eine Drehung des Umschalthebels in eine Position bewirkt, wobei der Druckabschnitt und der Umschalthebel starr miteinander verbunden sind, insbesondere ist das Umschaltelement einstückig gebildet. Eine Drehung des Umschalthebels führt somit zu einer Drehung des Druckabschnittes.

Insbesondere ist der Druckabschnitt derart ausgebildet, dass der Druckabschnitt in der erste Position des Umschaltelementes seitlich gegen das erste Sperrelement drückt, insbesondere drückt der Druckabschnitt auf die Innenseite des Verbindungsabschnitts des Sperrelementes. Dies führt dazu, dass das erste Sperrelement entgegen der Vorspannung von dem Zahnradabschnitt des Gelenkkopfes weggedrückt wird.

Ferner ist der Druckabschnitt derart ausgebildet, dass dieser in der zweiten Position des Umschaltelementes seitlich gegen das zweite Sperrelement drückt, und zwar bevorzugterweise ebenfalls gegen die Innenseite des zweiten Sperrelementes, sodass entgegen der jeweiligen Vorspannung dieses von dem Zahnradabschnitt des Gelenkkopfes weggedrückt wird.

Unter dem Begriff "Wegdrücken" ist insbesondere ein Schwenken zu verstehen, und zwar in Richtung der Innenwand des zweiten Abschnittes des Aufnahmebereiches. Dies führt dazu, dass das Sperrelement beabstandet vom Zahnradabschnitt des Gelenkkopfes angeordnet ist und somit keine Sperrfunktion ausbildet.

Ferner kann der Druckabschnitt derart ausgebildet sein, dass der Druckabschnitt in der dritten Position des Umschaltelementes seitlich gegen beide Sperrelemente drückt, sodass beide Sperrelement entgegen der jeweiligen Vorspannung von dem Zahnradabschnitt des Gelenkkopfes weggedrückt werden, sodass eine Freistellung ermöglicht wird. Insbesondere weist der Druckabschnitt die Form eines Kreissegmentes auf, in anderen Worten ist der Druckabschnitt fächerförmig gebildet. Der Druckabschnitt weist somit in Umfangsrichtung zwei radial äußere Enden auf, wobei die Enden zum seitlichen Drücken gegen jeweils ein Sperrelement dienen.

Dabei ist der Druckabschnitt derart ausgebildet, dass der Abstand zwischen den beiden radial äußeren Enden größer ausgebildet ist als der Abstand zwischen den Innenseiten der Verbindungsabschnitte der beiden Sperrelemente an der Stelle, an der der Druckabschnitt, bzw. dessen radial äußeren Enden, in einer dritten Position, angeordnet sind. Dies führt dazu, dass in der dritten Position jeweils ein radiales äußeres Ende gegen die Innenseite eines Sperrelementes stößt.

Insbesondere kann das Gelenk ein einziges Sperrelement mit einem ersten Zahnradabschnitt und einem zweiten Zahnradabschnitt umfassen. Insbesondere weist das Sperrelement ausschließlich den ersten und den zweiten Zahnradabschnitt auf. Es ist in der ersten Position des Umschaltelementes eine Drehbewegung des Gelenkkopfes im Aufnahmebereich des Gelenkkörpers in eine erste Richtung freigegeben und eine Drehbewegung in die entgegengesetzte zweite Richtung durch ein Eingreifen des ersten Zahnradabschnittes des Sperrelementes und des Zahnradabschnittes des Gelenkkopfes gesperrt, wobei in der zweiten Position eine Drehbewegung des Gelenkkopfes im Aufnahmebereich in die zweite Richtung freigegeben und eine Drehbewegung in die entgegengesetzte erste Richtung durch ein Eingreifen des zweiten Zahnradabschnittes des Sperrelementes und des Zahnradabschnittes des Gelenkkopfes gesperrt ist. Dabei kann in der dritten Position des Umschaltelementes kein Zahnradabschnitt des Sperrelementes im Eingriff mit dem Zahnradabschnitt des Gelenkkopfes stehen, sodass die Leerlauffunktion erreicht wird.

Insbesondere wird das Sperrelement durch eine Wippe gebildet. Ferner kann das Sperrelement durch einen sogenannten Umschaltnocken gebildet sein.

Das Sperrelement weist vor allem einen Umschalthebel auf, der seitlich vom Sperrelement hervorragt. Der Umschalthebel dient dazu, das Sperrelement in die verschiedenen Positionen und somit das Gelenk in die verschiedenen Funktionszustände zu bringen. Insbesondere ist das Sperrelement derart in den Aufnahmebereich des Gelenkkörpers eingebracht, dass der Umschalthebel seitlich aus einer entsprechenden Ausnehmung des Gelenkkörpers heraussteht und somit leicht zu betätigen ist. Das Sperrelement kann durch ein Vorspannelement vorgespannt sein, und zwar vorzugsweise derart, dass sich das Sperrelement, sollte es sich nicht in der ersten oder der zweiten Position befinden, stets in der dritten Position befindet. Das Vorspannelement kann insbesondere als Druckfeder ausgebildet sein.

Das Sperrelement kann auf einer ersten Seite bzw. Fläche die beiden Zahnradabschnitte aufweisen. Die erste Seite kann vor allem leicht gekrümmt sein. Auf einer gegenüberliegenden zweiten Seite bzw. Fläche kann das Sperrelement Vertiefungen aufweisen, die vorzugsweise kugelsegmentförmig oder kalottenförmig ausgebildet sind. Ferner kann zumindest ein Teil der zweiten Seite bzw. Fläche gewölbt ausgebildet sein. Das Gelenk kann ein Druckelement, vor allem ein federndes Druckstück aufweisen. Insbesondere umfasst das Druckelement eine Feder mit einer Arretierkugel, wobei die Arretierkugel in der Feder gehalten ist. Die Feder ist in dem Gelenkkörper, bspw. in einer Ausnehmung oder Bohrung, aufgenommen. Die Feder wird insbesondere stirnseitig auf die zweite Fläche des Sperrelementes gedrückt. Dabei kann das Sperrelement insbesondere pro Position mindestens eine Vertiefung bzw. Wölbung auf der zweiten Seite bzw. Fläche aufweisen, in der die Arretierkugel bei einem Wechsel der Position gleitet und das Sperrelement in der gewählten Position hält. In anderen Worten kann das Sperrelement drei Vertiefungen bzw. Wölbungen aufweisen.

Das Sperrelement weist vorzugsweise eine Schwenkachse auf und ist insbesondere konzentrisch in den zweiten Bereich der Ausnehmung des Gelenkkörpers eingebracht, sodass die Schwenkachse mittig angeordnet ist. Ausgehend von der zweiten Seite kann sich ferner der Umschalthebel, insbesondere in radialer Richtung in Bezug auf einer Schwenkachse des Sperrelementes, erstrecken. Das Sperrelement und der Umschalthebel sind insbesondere einstückig ausgebildet. Ferner können das Sperrelement und das Umschaltelement zwei Bauteile darstellen, die insbesondere über eine formschlüssige Verbindung in Eingriff miteinander stehen.

Insbesondere weist das Sperrelement eine nahezu kreisförmige Form in Draufsicht auf, die jedoch an einer Seite abgeflacht ist. An dieser Seite, die sich gegenüber der Seite befindet, auf der der Umschalthebel angeordnet ist, ist in Draufsicht seitlich jeweils ein Zahnradabschnitt angeordnet. Insbesondere weist jeder Zahnradabschnitt zwischen 1 und 6, insbesondere zwischen 2 und 4 Zähne auf.

Dadurch dass die beiden Zahnradabschnitte auf einer abgeflachten Seite des Sperrelementes angeordnet sind, sind sie in Bezug zur Schwenkachse des Sperrelementes nicht in Umfangrichtung, d.h. tangential, verlaufend ausgebildet, sondern sie verlaufen in einem Winkel zur tangentialen Richtung. Der Winkel beträgt vor allem zwischen 30° und 50°. Die Sperrfunktion liegt daran, dass die Zahnradabschnitt nicht tangential in Bezug zur Schwenkachse angeordnet sind, sodass die Zähne bei einer Drehung in eine Richtung auseinandergleiten, d.h. ratschen, während sich bei einer Drehung in die andere Richtung, die Zähne verkanten.

Insgesamt ist das Sperrelement somit zylinderförmig ausgebildet, bis auf den Umschalthebel, der radial hervorsteht, und den abgeflachten Bereich mit den Zahnradabschnitten.

Das Sperrelement oder das Umschaltelement ist in einer Position vorzugsweise durch mindestens ein Druckelement fixierbar. Das Druckelement ist vor allem als federndes Druckstück, vor allem Feder, mit einer Arretierkugel ausgebildet. Die Arretierkugel ist in der Feder gehalten. Dabei kann das Sperrelement oder das Umschaltelement pro Position mindestens eine Vertiefung, vorzugsweise pro Position genau eine oder zwei Vertiefungen, aufweisen, in der die Arretierkugel bei einem Wechsel der Position gleitet und das Sperrelement oder das Umschaltelement in der gewählten Position hält. Die Vertiefungen sind insbesondere auf einer Kreislinie angeordnet.

Das Druckelement kann dabei insbesondere derart angeordnet sein, dass die Arretierkugel bei einer Bewegung des Umschaltelementes bzw. der Sperrelementes entlang einer Fläche des Umschaltelementes bewegen kann.

Ferner kann der Gelenkkörper auf einer Innenseite, insbesondere kugelsegementförmige oder kalottenförmige, Vertiefungen aufweisen, wobei das federnde Druckstück derart angeordnet ist, dass die Arretierkugel je nach Position des Sperrelementes in eine Vertiefung des Gelenkkörpers gleitet und das Sperrelement in der Position hält. Dabei kann das federnde Druckstück mit dem Sperrelement verbunden sein. Bspw. kann das federnde Druckstück mit einem Ende in eine Ausnehmung des Sperrelement eingebracht sein, sodass sich dieses mit dem Sperrelement mit bewegt. Dabei steht es vor allem in Richtung der Innenseite des Gelenkkörpers, der die Vertiefungen aufweist, vorm Sperrelement ab.

Ferner bevorzugt weist das Gelenk mindestens ein Gleitelement auf, wobei das Gleitelement selbstschmierend ausgebildet ist. Das Gleitelement kann insbesondere als Gleitbüchse oder Gleitscheibe ausgebildet sein. Das Gleitelement dient insbesondere zur Flächenlagerung oder zur Achslagerung und somit der Verbesserung der Laufeigenschaften des Gelenkes. Insbesondere ist das mindestens eine Gleitelement aus Messing oder Polytetrafluorethylen, in anderen Worten Teflon, ausgebildet. Dabei dient Polytetrafluorethylen vor allem für die Flächenlagerung, allerdings nicht für die Achslagerung.

In einem weiteren Aspekt umfasst die vorliegende Erfindung eine orthopädische Quengelschiene umfassend mindestens ein oben beschriebenes Gelenk, wobei die orthopädische Quengelschiene zwei Schienenelemente umfasst, wobei ein erstes Schienenelement starr mit dem Gelenkkörper verbunden ist, und wobei ein zweites Schienenelement starr mit dem Gelenkkopf verbunden ist. Insbesondere handelt es sich bei der Quengelschiene um eine Gelenkschiene, insbesondere eine statische Gelenkschiene. In der Umgangssprache wird auch die Quengelschiene als Redressionsgelenk oder Quengelgelenk betitelt. Das Quengelgelenk ist insbesondere kombinierbar mit anderen Schienen beziehungsweise um entsprechende Funktionen erweiterbar. Ferner kann die Quengelschiene zwei oben beschriebene Gelenke umfassen, sodass ein Doppelgelenk vorliegt, das die Redression in mehreren Bewegungsebenen ermöglicht. Das Gleitelement ist insbesondere zwischen den Schienenelementen angeordnet.

### Kurze Beschreibung der Zeichnungen

Die Erfindung wird anhand der folgenden rein schematischen Figuren näher erläutert.

Die Figuren zeigen:
- Figur 1: eine Draufsicht auf ein erfindungsgemäßes Gelenk;
- Figur 2: eine weitere Draufsicht auf das Gelenk der Figur 1;
- Figur 3:: eine weitere Draufsicht auf das Gelenk der Figuren 1 und 2;
- Figur 4:: eine Draufsicht auf die andere Seite des Gelenks der Figuren 1 bis 3;
- Figur 5:: eine Draufsicht auf eine Quengelschiene umfassend ein erfindungsgemäßes Gelenk nach den Figuren 1 bis 4; und
- Figur 6:: eine weitere Draufsicht auf die Quengelschiene der Figur 5;
- Figur 7:: eine Draufsicht auf ein erfindungsgemäßes Gelenk;
- Figur 8:: eine Draufsicht auf das Gelenk der Figur 7;
- Figur 9:: eine Draufsicht auf den Gelenkkörper des Gelenks der Figuren 7 und 8; und
- Figur 10:: eine Draufsicht auf das Sperrelement des Gelenks der Figuren 7 bis 9; und
- Figur 11:: eine Explosionsdarstellung einer Quengelschiene umfassend ein erfindungsgemäßes Gelenk nach den Figuren 7 bis 10.

### Ausführungsformen der Erfindung

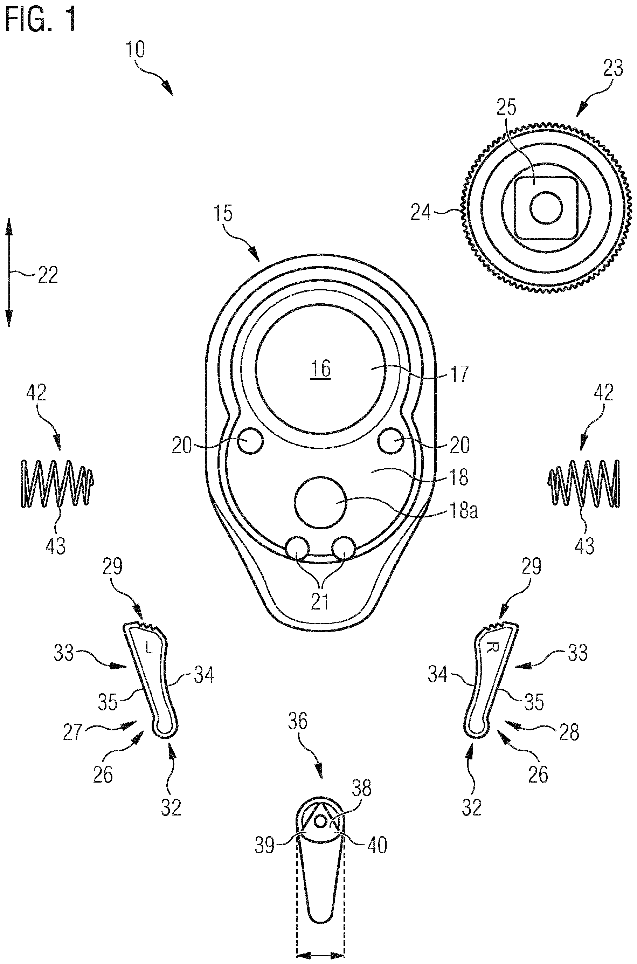

Figur 1 zeigt eine Draufsicht auf ein erfindungsgemäßes Gelenk 10, umfassend einen Gelenkkörper 15, einen Gelenkkopf 23, zwei Sperrelemente 26 mit jeweils einer Schwenkachse 26a, und zwar ein erstes Sperrelement 27 und ein zweites Sperrelement 28, ein Umschaltelement 36 mit einer Schwenkachse 36a und zwei Vorspannelemente 42.

Der Gelenkkopf 23 mit der Schwenkachse 23a weist einen Zahnradabschnitt 24 auf, der sich vollumfänglich um den zylinderförmigen Gelenkkopf 23 erstreckt. Der Zahnradabschnitt 24 ist als Außenverzahnung ausgebildet. Ferner weist der Gelenkkopf einen Befestigungsabschnitt 25 auf, der stirnseitig hervorsteht.

Dieser Befestigungsabschnitt 25 ist in Form eines Vierkants ausgebildet. Er dient zur Befestigung mit einem Schienenelement einer Quengelschiene.

Der Gelenkkörper 15 weist einen Aufnahmebereich 16 auf, der in Längsrichtung 22 des Gelenkkörpers achtförmig ausgebildet ist. In anderen Worten weist der Aufnahmebereich 16 einen ersten Bereich 17 und einen zweiten Bereich 18 auf. Der erste Bereich 17 dient zur Aufnahme des Gelenkkopfes 23, während der zweite Bereich 18 zur Aufnahme der Sperrelemente 26, des Umschaltelementes 36 und der Vorspannelemente 42 dient.

Ferner weist der Gelenkkörper eine Innenwand 19 auf, die den Aufnahmebereich 16 begrenzt. Im ersten Bereich 17 des Aufnahmebereiches 16 ist eine erste, kreisförmige Durchbrechung 17a ausgebildet. Der Gelenkkopf 23 ist derart in den ersten Bereich 17 des Aufnahmebereichs 16 eingebracht wird, dass der Befestigungsabschnitt 25 aus dem Aufnahmebereich 16 hervorragt.

Ferner weist der Gelenkkörper 15 innerhalb des Aufnahmebereichs 16 Befestigungsbohrungen auf, die dazu dienen, den Gelenkkörper auf der hier nicht dargestellten Rückseite mit einem Schienenelement zu verbinden. Ferner weist der Gelenkkörper zwei Ausnehmungen 21 auf, die als Sackbohrungen ausgebildet sind und zur Aufnahme der Sperrelemente 26 ausgebildet sind.

Die Sperrelemente 26 weisen jeweils einen Zahnradabschnitt 29 auf und einen Befestigungsabschnitt 32. Dazwischen ist ein Verbindungsabschnitt 33 angeordnet. Der Verbindungsabschnitt 33 weist eine Außenseite 35 und eine Innenseite 34 auf. Der Zahnradabschnitt 29 steht insbesondere in einem Winkel von 20° bis 40° zur Längsrichtung des Sperrelementes.

Das Umschaltelement 36 weist einen Umschalthebel 37 auf, der in der Perspektive der Figur 1 nicht zu sehen ist, da er sich auf der nicht gezeigten Seite des Umschaltelementes 36 befindet. Ferner weist das Umschaltelement 36 einen Druckabschnitt 38 auf, der die Form eines Kreissegmentes aufweist.

Daher weist der Druckabschnitt ein erstes radial äußeres Ende 39 und ein zweites radial äußeres Ende 40 in Umfangsrichtung auf.

Die Vorspannelemente 42 sind jeweils als Druckfeder 43 ausgebildet. Das Umschaltelement 36 ist derart in die zweite Durchbrechung 18a eingebracht, dass sich der Umschalthebel 37 auf der nicht gezeigten Seite des Gelenkkörpers 15 befindet, während sich der Druckabschnitt 38 in dem Aufnahmebereich 16 befindet.

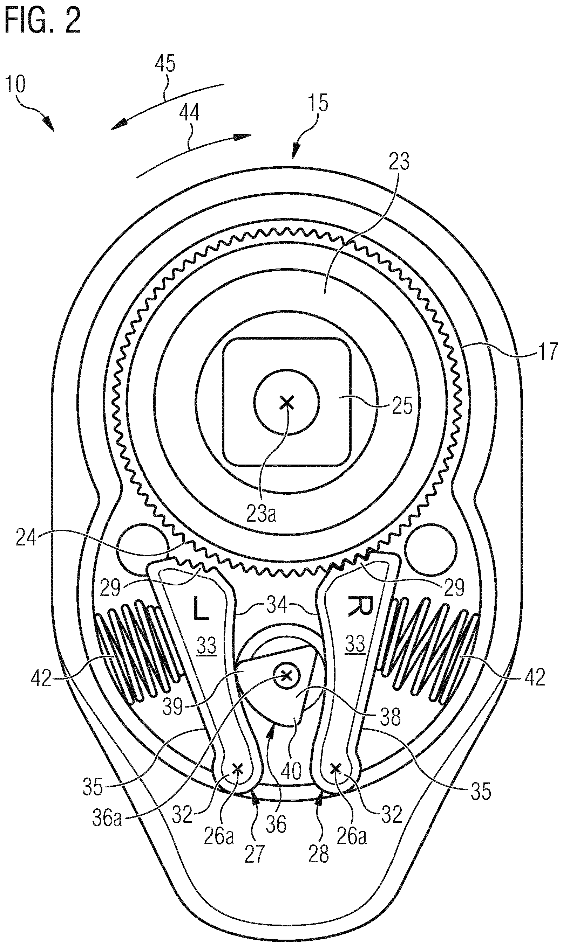

Figur 2 zeigt eine weitere Draufsicht auf das Gelenk 10 der Figur 1 in einer zweiten Position des Umschaltelementes 36, wobei die zuvor beschriebenen Elemente der Figur 1 in den Aufnahmebereich 16 des Gelenkkörpers 15 eingebracht sind. Die Sperrelemente sind mit ihrem jeweiligen Befestigungsabschnitt in die Ausnehmungen 21 des Gelenkkörpers 15 eingebracht, und können um eine dort angeordnete Schwenkachse verschwenkt werden. Dabei drückt gegen die Außenseite 35 des Verbindungsabschnittes 33 jeweils ein Vorspannelement 42. Das linke Vorspannelement 42, das seitlich gegen das erste Sperrelemente 27 drückt, würde somit eine Schwenkung des ersten Sperrelementes 27 im Uhrzeigersinn bewirken. Andersherum würde das Vorspannelement 42, das gegen das zweite Sperrelemente seitlich drückt, eine Schwenkung entgegen der Uhrzeigerrichtung bewirken.

Der Gelenkkopf 23 ist derart in den Aufnahmebereich 16, und zwar in den ersten Bereich 17 aufgenommen, dass die Zahnradabschnitte 29 der Sperrelemente 26 gegen den Zahnradabschnitt 24 des Gelenkkopfes 23 stoßen und somit mittels der Vorspannelemente die Zahnradabschnitte 29 gegen den Zahnradabschnitt 24 des Gelenkkopfes 23 gedrückt werden. Durch das Drücken gegen den Zahnradabschnitt 24 des Gelenkkopfes 23 wird der Gelenkkopf 23 in seiner Drehbewegung gesperrt. Das Umschaltelement 36, und zwar dessen Druckabschnitt 38, ist zwischen dem ersten Sperrelement 27 und dem zweiten Sperrelement 28 angeordnet.

Dabei ist das Umschaltelement in eine zweite Position gebracht, sodass die Drehbewegung des Gelenkkopfes 23 in zweiter Richtung 45 freigegeben ist und in erster Richtung 44 gesperrt ist. Dies wird dadurch ermöglicht, dass der Druckabschnitt 38, nämlich das erste Ende 39 gegen die Innenseite 34 des Verbindungsabschnittes 33 des ersten Sperrelementes 27 drückt. Somit wird dessen Zahnradabschnitt 39 vom Zahnradabschnitt 24 des Gelenkkopfes 23 weggedrückt und kann somit seine Sperrfunktion, und zwar eine Blockade der Drehbewegung in zweiter Richtung 45, nicht wahrnehmen. Durch die schräge Position des Sperrelementes in Bezug auf den Zahnradabschnitt 24 des Gelenkkopfes 23 wird eine Drehbewegung in zweiter Richtung 45 durch das zweite Sperrelement 28 nicht gesperrt.

Analog wird in einer ersten Position das zweite Ende 40 des Druckabschnittes 38 gegen die Innenseite 34 des Verbindungsabschnitts 33 des zweiten Sperrelementes 28 gedrückt und bewegt dieses vom Zahnradabschnitt 24 des Gelenkkopfes 23 weg, während die Zahnradabschnitte 29 des ersten Sperrelementes 27 im Eingriff mit dem Zahnradabschnitt 24 steht, sodass eine Bewegung in erster Richtung 44 des Gelenkkopfes 23 ermöglicht wird, jedoch eine Drehbewegung in die zweite Richtung 45 gesperrt ist.

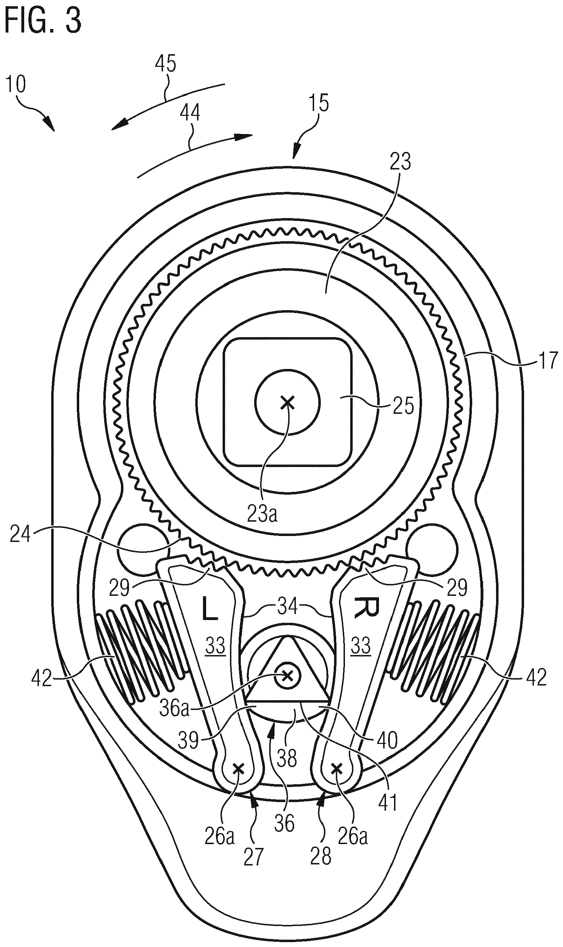

Figur 3 zeigt ist eine weitere Draufsicht auf das Gelenk 10 der Figuren 1 und 2 in einer dritten Position des Umschaltelementes 36.

In der dritten Position drückt sowohl das erste Ende 39 des Druckabschnitts 38 gegen die Innenseite 34 des ersten Sperrelementes 27 als auch das zweite Ende 40 gegen die Innenseite 34 des zweiten Sperrelementes 28, sodass sowohl das erste Sperrelement 27 als auch das zweite Sperrelement 28 von dem Zahnradabschnitt 24 des Gelenkkopfes 23 weggedrückt werden. Insgesamt ist somit eine Drehbewegung des Gelenkkopfes 23 sowohl in erster Richtung 44 als auch in zweiter Richtung 45 möglich. Dazu ist der Druckabschnitt 38 des Umschaltelementes 36 derart ausgebildet, dass eine gradlinige Verbindungslinie 41 zwischen dem ersten Ende 39 und dem zweiten Ende 40 größer ist als der entsprechende Abstand der Innenseiten 34 der beiden Sperrelemente auf derselben Höhe, sodass der Druckabschnitt 38 dazu ausgebildet ist, beide Sperrelemente gleichzeitig von dem Zahnradabschnitt 24 wegzudrücken, und zwar entgegen der Vorspannkraft der Vorspannelemente 42 in Richtung der Innenwand 19.

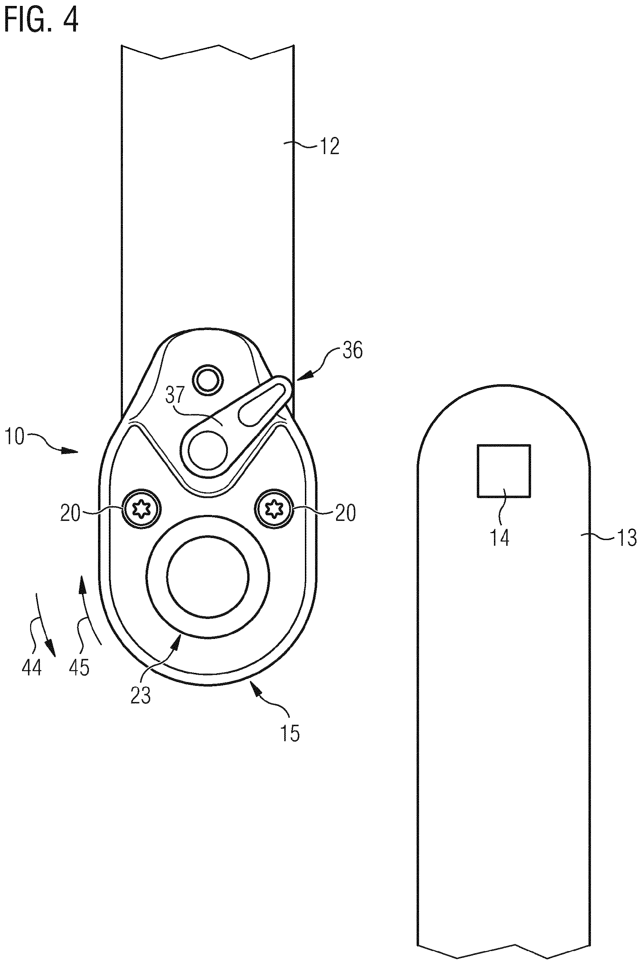

In Figur 4 ist eine Draufsicht auf die andere Seite des Gelenkes 10 sowie auf zwei Schienenelemente einer Quengelschiene 11 gezeigt. Und zwar ist das Gelenk in der ersten Position des Umschaltelementes 36 gezeigt. Ferner ist der Umschalthebel 37 gezeigt, wie dieser in einer zweiten Position angeordnet ist. Ferner sind mittels der entsprechenden Befestigungsbohrungen 20 das Gelenk 10 an einem ersten Schienenelement 12 starr befestigt. Ferner ist ein zweites Schienenelement 13 gezeigt, das eine Durchbrechung 14 aufweist zur Verbindung mit dem Befestigungsabschnitt 25 des Gelenkkopfes 23.

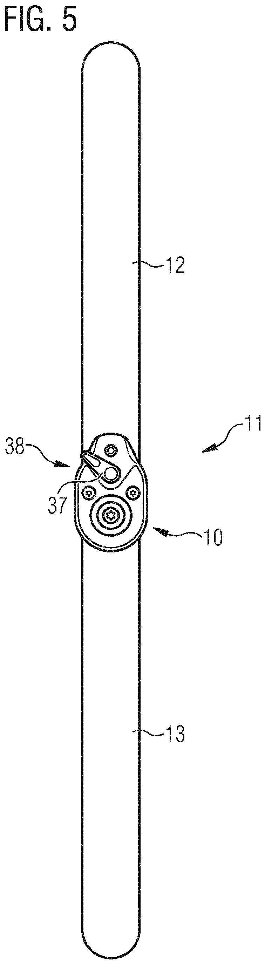

Figur 5 zeigt eine Draufsicht auf eine Quengelschiene 11 umfassend ein erfindungsgemäßes Gelenk 10 nach den Figuren 1 bis 4, wobei nun das Gelenk 10 starr mit dem zweiten Schienenelement 13 verbunden ist und wobei der Umschalthebel in einer zweiten Position gezeigt ist.

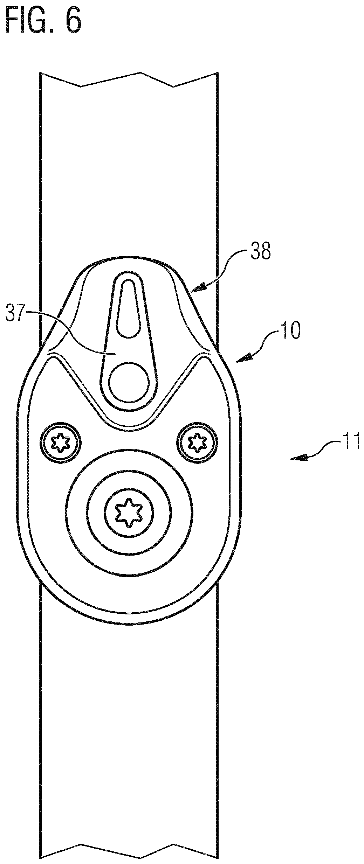

Figur 6 zeigt eine weitere Draufsicht auf die Quengelschiene 11 der Figur 5, wobei der Umschalthebel 37 des Umschaltelementes 38 in einer dritten Position dargestellt ist, in der Drehbewegungen des Gelenkkopfes in die erste Richtung 44 und die zweite Richtung 45 möglich sind.

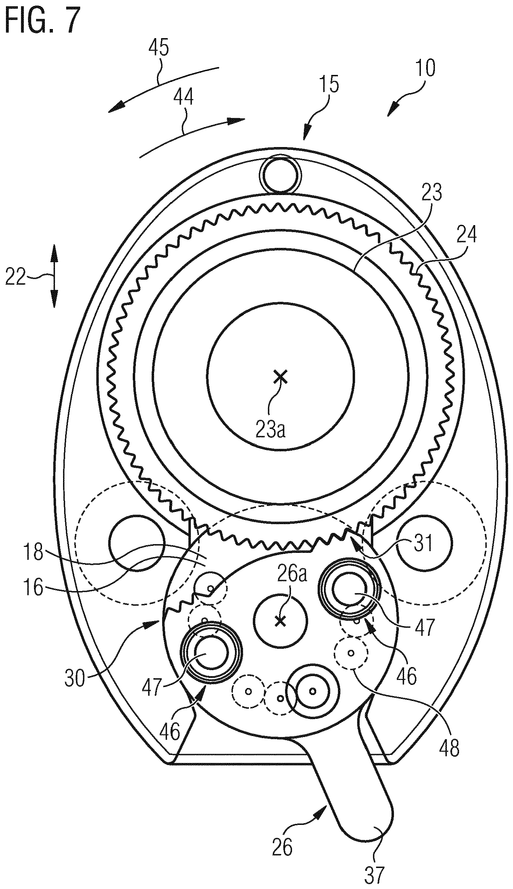

Figur 7 zeigt eine Draufsicht auf ein erfindungsgemäßes Gelenk 10 umfassend einen Gelenkkörper 15, einen Gelenkkopf 23 mit einer Schwenkachse 23a und ein einziges Sperrelement 26 mit einer Schwenkachse 26a.

Der Gelenkkörper 15 umfasst einen Aufnahmebereich 16, der in Längsrichtung 22 des Gelenkkörpers 15 achtförmig ausgebildet ist. Der Aufnahmebereich 16 weist somit einen ersten Bereich 17 auf, in den der Gelenkkopf 23 eingebracht ist, und einen zweiten Bereich 18, in den das Sperrelement 26 eingebracht ist.

Der Gelenkkopf 23 ist konzentrisch im ersten Bereich 17 angeordnet, während das Sperrelement konzentrisch im zweiten Bereich 18 angeordnet ist.

Der Gelenkkopf 23 weist einen Zahnradabschnitt 24 auf, der sich vollumfänglich um den zylinderförmigen Gelenkkopf erstreckt. Der Zahnradabschnitt 24 ist als Außenverzahnung ausgebildet.

Das Sperrelement 26 weist einen Umschalthebel 37 auf, mit der es in die verschiedenen Positionen bringbar ist. Der Umschalthebel 37 ragt seitlich aus dem Aufnahmebereich 16 des Gelenkkörpers 15 heraus.

Das Sperrelement 26 weist einen ersten Zahnradabschnitt 30 und einen zweiten Zahnradabschnitt 31 auf. Allerdings sind die beiden Zahnradabschnitte auf einer abgeflachten Seite des Sperrelementes 26 angeordnet, sodass sie in Bezug auf die Schwenkachse 26a des Sperrelementes nicht in Umfangrichtung, d.h. tangential, verlaufen, sondern in einem Winkel zur tangentialen Richtung. Bis auf die abgeflachte Seite und den Umschalthebel 37 weist das Sperrelement an sich eine zylinderförmige Form auf.

Das Sperrelement 26 ist in eine zweite Position gebracht, sodass der zweite Zahnradabschnitt 31 in den Zahnradabschnitt 24 des Gelenkkopfes 23 greift und eine Drehbewegung in die erste Richtung 44 sperrt, während eine Drehbewegung in die zweite Richtung 45 freigegeben ist. Die Sperrfunktion liegt daran, dass die Zahnradabschnitt 30, 31 nicht tangential in Bezug auf die Schwenkachse 26a angeordnet sind, sodass die Zähne bei einer Drehung in zweiter Richtung auseinandergleiten, d.h. ratschen, während sich die Zähne bei einer Drehung in zweiter Richtung verkanten.

Zur Fixierung einer der Positionen weist das Sperrelement 26 zwei federnde Druckstücke 46 auf, die jeweils eine Arretierkugel 47 umfassen. Die Arretierkugel 47 gleitet über eine stirnseitige Innenseite 15a des Gelenkkörpers 15, in der Vertiefungen 48 eingebracht sind, und zwar mindestens eine Vertiefung pro Position und federndem Druckstück 46. Die Vertiefungen 47 sind auf einer Kreislinie angeordnet, auf der sich die Arretierkugel 47 bewegt und somit das Sperrelement 26 in einer Position halten kann. Ferner kann die Arretierkugel 47 bei entsprechender Betätigung des Umschalthebels 37 aus einer Position herausgleiten und in eine neue Position gleiten.

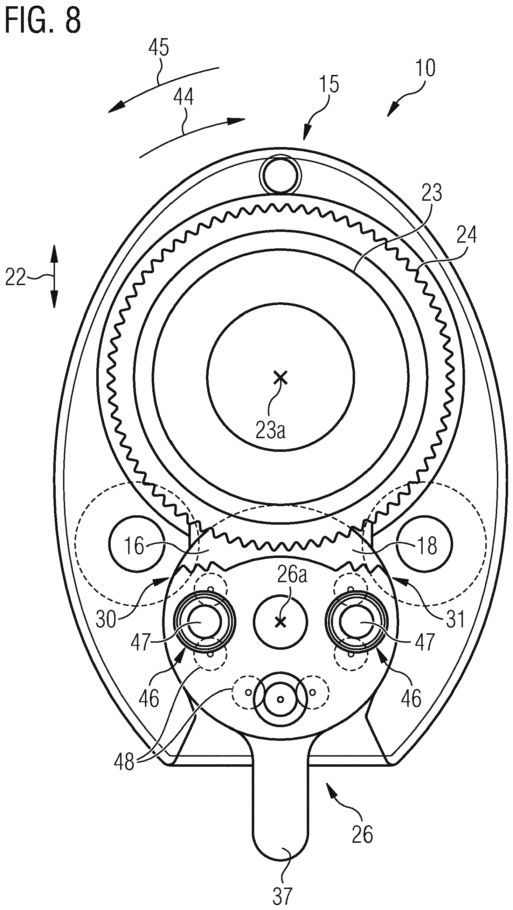

In Figur 8 ist eine Draufsicht auf das Gelenk 10 der Figur 7 zu sehen, in der das Sperrelement 26 in der dritten Position angeordnet ist, sodass keiner der Zahnradabschnitte 30, 31 in Eingriff mit dem Zahnradabschnitt 24 des Gelenkkopfes 23 steht. Somit ist die Leerlauffunktion gewährleistet und eine Drehbewegung sowohl in erster Richtung 44 als auch in zweiter Richtung 45 des Gelenkkopfes 23 möglich. Der Gelenkkopf 23 und das Sperrelement 26 sind somit derart zueinander angeordnet sind, dass in der zweiten und in der ersten Position des Sperrelementes 26 jeweils ein Zahnradabschnitt des Sperrelementes 26 in den Zahnradabschnitt 24 des Gelenkkopfes greift, während in der dritten Position (siehe Figur 8) beide Zahnradabschnitte des Sperrelementes 26 vom Zahnradabschnitt 24 des Gelenkkopfes 23 beabstandet sind.

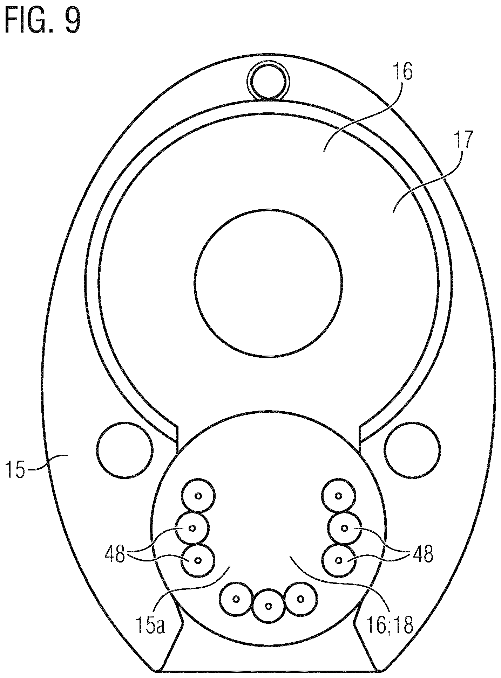

Figur 9 zeigt eine Draufsicht auf den Gelenkkörper 15 des Gelenks 10 der Figuren 7 und 8, die eine bessere Sicht auf die stirnseitige Innenfläche 15a zeigt, die die Ausnehmung 16 beziehungsweise dessen zweiten Bereich 18 in axialer Richtung begrenzt. Die Innenseite 15a umfasst Vertiefungen 48 für die Arretierkugeln 47 der Druckstücke 46.

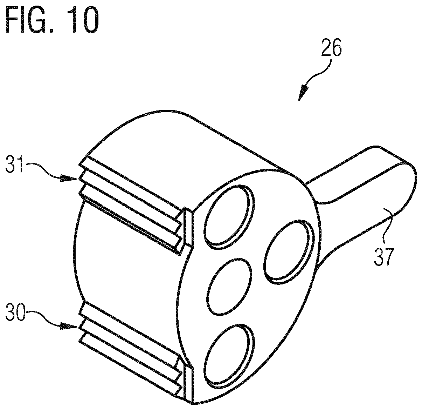

In Figur 10 ist eine Draufsicht auf das Sperrelement 26 des Gelenks 10 der Figuren 7 bis 9 zeigt, wobei deutlich zu sehen ist, wie der Umschalthebel seitlich hervorragt und wie auf einer abgeflachten gegenüberliegenden Seite des Sperrelementes 26 die Zahnradabschnitte 30, 31 angeordnet sind. Bis auf die abgeflachte Seite und den Umschalthebel 37 weist das Sperrelement an sich eine zylinderförmige Form auf.

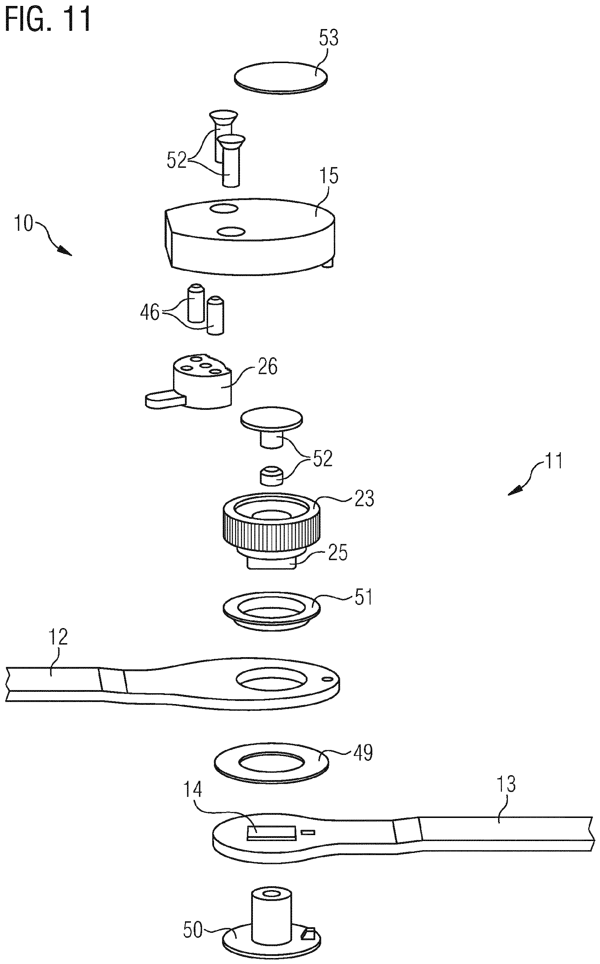

In Figur 11 ist eine Explosionsdarstellung einer Quengelschiene 11 umfassend ein erfindungsgemäßes Gelenk 10 nach den Figuren 7 bis 9 gezeigt.

Die Quengelschiene 11 umfasst ein erstes Schienenelement 12 und ein zweites Schienenelement 13. Das zweite Schienenelement 13 weist eine Durchbrechung 14 auf, in den der vierkantförmige Befestigungsabschnitt 25 des Gelenkkopfes greifen kann.

Ferner weist die Quengelschiene 11 ein Gleitelement 49, und zwar eine Gleitscheibe, auf, die zwischen dem ersten Schienenelement 12 und dem zweiten Schienenelement 13 angeordnet ist. Das zweite Schienenelement 13 ist mittels einer Achse 50 und einer Bundbuchse 51 sowie Schrauben 52 mit dem Gelenkkopf 23 verbunden.

Dabei ist die Bundbuchse 51 derart in eine Ausnehmung des ersten Schienenelementes 12 eingebracht, dass eine Bewegung des zweiten Schienenelementes 13 gegenüber dem ersten Schienenelement 12 und dem damit starr verbundenen Gelenkkopf 23 möglich ist. Ferner ist in Figur 10 das Sperrelement und zwei federnde Druckstücke 46 sowie der Gelenkkörper 15 des Gelenks 10 zu sehen. Zudem weist das Quengelgelenk 11 noch weitere Schrauben 52 und eine Ronde 53 auf.

### Bezugszeichenliste

- 10: Gelenk
- 11: Quengelschiene
- 12: erstes Schienenelement
- 13: zweites Schienenelement
- 14: Durchbrechung
- 15: Gelenkkörper
- 15a: Innenseite
- 16: Aufnahmebereich
- 17: erster Bereich
- 17a: erste Durchbrechung
- 18: zweiter Bereich
- 18a: zweite Durchbrechung
- 19: Innenwand
- 20: Befestigungsbohrung
- 21: Ausnehmungen zur Aufnahme der Sperrelemente
- 22: Längsrichtung
- 23: Gelenkkopf
- 23a: Schwenkachse
- 24: Zahnradabschnitt
- 25: Befestigungsabschnitt
- 26: Sperrelement
- 26a: Schwenkachse
- 27: erstes Sperrelement
- 28: zweites Sperrelement
- 29: Zahnradabschnitt
- 30: erster Zahnradabschnitt
- 31: zweiter Zahnradabschnitt
- 32: Befestigungsabschnitt
- 33: Verbindungabschnitt
- 34: Innenseite
- 35: Außenseite
- 36: Umschaltelement
- 36a: Schwenkachse36
- 37: Umschalthebel
- 38: Druckabschnitt
- 39: erstes Ende
- 40: zweites Ende
- 41: Verbindungslinie
- 42: Vorspannelement
- 43: Druckfeder
- 44: erste Richtung
- 45: zweite Richtung
- 46: federndes Druckstück
- 47: Arretierkugel
- 48: Vertiefung
- 49: Gleitelement
- 50: Achse
- 51: Bundbuchse
- 52: Schraube
- 53: Ronde

## Patentansprüche

1. Gelenk (10) für eine orthopädische Quengelschiene (11),
wobei das Gelenk (10) einen Gelenkkopf (23) mit einem Zahnradabschnitt (24), einen Gelenkkörper (15) und ein Umschaltelement (36) und ein erstes Sperrelement (27) und ein zweites Sperrelement (28) umfasst, wobei das erste Sperrelement (27) und das zweite Sperrelement (28) jeweils einen Zahnradabschnitt (29) aufweisen,
wobei der Gelenkkörper (15) einen Aufnahmebereich (16) aufweist, wobei der Gelenkkopf (23) drehbar im Aufnahmebereich (16) angeordnet ist,
wobei die Sperrelemente schwenkbar im Aufnahmebereich (16) angeordnet sind, und
wobei das Umschaltelement (36) in mindestens eine erste Position und eine zweite Position bringbar ist,
wobei in der ersten Position des Umschaltelements (36) eine Drehbewegung des Gelenkkopfes (23) im Aufnahmebereich (16) in die erste Richtung (44) freigegeben und eine Drehbewegung in die entgegengesetzte zweite Richtung (45) durch ein Eingreifen des Zahnradabschnitts (29) des ersten Sperrelementes (27) und des Zahnradabschnittes (24) des Gelenkkopfes (23) gesperrt ist, wobei in der zweiten Position des Umschaltelements eine Drehbewegung des Gelenkkopfes (23) im Aufnahmebereich (16l) in die zweite Richtung (45) freigegeben und eine Drehbewegung in die entgegensetzte erste Richtung (44) durch ein Eingreifen des Zahnradabschnitts (29) des zweiten Sperrelementes (2) und des Zahnradabschnittes (24) des Gelenkkopfes (23) gesperrt ist.

2. Gelenk (10) nach Anspruch 1,
wobei,
das Umschaltelement (36) in eine dritte Position bringbar ist,
wobei in der dritten Position eine Drehbewegung des Gelenkkopfes (23) im Aufnahmebereich (16) des Gelenkkörpers (15) in erster Richtung (44) und zweiter Richtung (45) freigegeben ist.

3. Gelenk (10) nach einem der vorherigen Ansprüche,
wobei mindestens ein Sperrelement (26) durch mindestens ein Vorspannelement (42) vorgespannt ist.

4. Gelenk (10) nach einem der vorherigen Ansprüche,
wobei das erste Sperrelement (27) und das zweite Sperrelement (28) jeweils durch ein Vorspannelement (42) vorgespannt sind.

5. Gelenk (10) nach Anspruch 4,
wobei
das Umschaltelement (36) einen Druckabschnitt (38) aufweist, wobei der Druckabschnitt (38) zwischen dem ersten Sperrelement (27) und dem zweiten Sperrelement (28) angeordnet ist,
wobei der Druckabschnitt (38) derart ausgebildet ist, dass der Druckabschnitt (38) in der ersten Position des Umschaltelementes (36) seitlich gegen das erste Sperrelement (27) drückt, sodass das erste Sperrelement (27) entgegen der Vorspannung von dem Zahnradabschnitt (24) des Gelenkkopfes (23) weggedrückt wird,
und wobei der Druckabschnitt (38) derart ausgebildet ist, dass der Druckabschnitt (38) in der zweiten Position des Umschaltelementes (36) seitlich gegen das zweite Sperrelement (28) drückt, sodass das zweite Sperrelement (28) entgegen der Vorspannung von dem Zahnradabschnitt (24) des Gelenkkopfes (23) weggedrückt wird.

6. Gelenk (10) nach Anspruch 5,
wobei das Umschaltelement (36) in eine dritte Position bringbar ist und der Druckabschnitt (38) derart ausgebildet ist, dass der Druckabschnitt (38) in der dritten Position des Umschaltelementes (36) seitlich gegen beide Sperrelemente (26) drückt,
sodass das beide Sperrelemente (26) entgegen der jeweiligen Vorspannung von dem Zahnradabschnitt (24) des Gelenkkopfes (23) weggedrückt werden.

7. Gelenk (10) nach einem der Ansprüche 5 oder 6,
wobei
der Druckabschnitt (38) die Form eines Kreissegments aufweist, sodass der Druckabschnitt (38) in Umfangsrichtung zwei radial äußere Enden aufweist, wobei die Enden zum seitlichen Drücken gegen jeweils ein Sperrelement (26) dienen.

8. Gelenk (10) für eine orthopädische Quengelschiene (11),
wobei
das Gelenk einen Gelenkkopf (23) mit einem Zahnradabschnitt (24), einen Gelenkkörper (15) und ein einziges Sperrelement (26) mit einem ersten Zahnradabschnitts (30) und einem zweiten Zahnradabschnitt (31) aufweist, wobei der Gelenkkörper (15) einen Aufnahmebereich (16) aufweist, wobei der Gelenkkopf (23) drehbar im Aufnahmebereit (16) angeordnet ist, wobei das Sperrelement (26) schwenkbar im Aufnahmebereich (16) angeordnet ist, und wobei das Sperrelement (26) in mindestens eine erste Position und eine zweite Position bringbar ist,
wobei in der ersten Position des Sperrelementes (26) eine Drehbewegung des Gelenkkopfes (23) im Aufnahmebereich (16) in die erste Richtung (44) freigegeben und eine Drehbewegung in die entgegensetzte zweite Richtung (45) durch ein Eingreifen des ersten Zahnradabschnitts (30) des Sperrelements (26) und des Zahnradabschnitts (24) des Gelenkkopfes (23) gesperrt ist,
und wobei in der zweiten Position eine Drehbewegung des Gelenkkopfes (23) im Aufnahmebereich (16) in die zweite Richtung (45) freigegeben und eine Drehbewegung in die entgegensetzte erste Richtung (44) durch ein Eingreifen des zweiten Zahnradabschnitts (31) des Sperrelements (26) und des Zahnradabschnitts (24) des Gelenkkopfes (23) gesperrt ist.

9. Gelenk (10) nach Anspruch 8,
wobei das Sperrelement (26) in eine dritte Position bringbar ist, wobei in der dritten Position des Sperrelementes (26) kein Zahnradabschnitt des Sperrelementes (26) im Eingriff mit dem Zahnradabschnitt (24) des Gelenkkopfes (23) steht.

10. Gelenk (10) nach einem der Ansprüche 8 oder 9,
wobei
das Sperrelement (26) einen Umschalthebel (37) aufweist.

11. Gelenk (10) nach einem der vorherigen Ansprüche,
wobei
das einzige Sperrelement (26) oder das Umschaltelement (36) in einer Position durch mindestens ein Druckelement fixierbar ist.

12. Gelenk (10) nach Anspruch 11,
wobei
das Druckelement als federndes Druckstück (46) mit einer Arretierkugel (47) ausgebildet ist.

13. Gelenk (10) nach Anspruch 12,
wobei
der Gelenkkörper (15) auf einer Innenseite (15a) Vertiefungen (48) aufweist,
wobei das federnde Druckstück (46) derart angeordnet ist, dass die Arretierkugel (47) je nach Position des Sperrelementes (26) in eine Vertiefung (48) des Gelenkkörpers (15) gleitet um das Sperrelement (26) in der Position zu halten.

14. Orthopädische Quengelschiene umfassend mindestens ein Gelenk nach einem der Ansprüche 1 bis 13,
wobei die orthopädische Quengelschiene zwei Schienenelemente umfasst, wobei ein erstes Schienenelement (12) starr mit dem Gelenkkörper (15) verbunden ist, und wobei ein zweites Schienenelement (13) starr mit dem Gelenkkopf (23) verbunden ist.

## Claims

1. Joint (10) for an orthopedic Quengel splint (11),
wherein
the joint (10) comprises a joint head (23) with a gear wheel section (24), a joint body (15) and a switching element (36) and a first blocking element (27) and a second blocking element (28), wherein the first blocking element (27) and the second blocking element (28) each have one gear wheel section (29),
wherein the joint body (15) has a receiving area (16),
wherein the joint head (23) is rotatably arranged in the receiving area (16), wherein the blocking elements are arranged pivotably in the receiving area (16), and
wherein the switching element (36) can be brought into at least a first position and a second position,
wherein in the first position of the switching element (36) a rotational movement of the joint head (23) in the receiving area (16) is released in the first direction (44) and a rotational movement in the opposite second direction (45) is blocked by the engagement of the gear wheel section (29) of the first blocking element (27) and the gear wheel section (24) of the joint head (23), wherein in the second position of the switching element a rotational movement of the joint head (23) in the receiving area (16) is released in the second direction (45) and a rotational movement in the opposite first direction (44) is blocked by the engagement of a gear wheel section (29) of the second blocking element (28) and the gear wheel section (24) of the joint head (23).

2. Joint (10) according to claim 1,
wherein
the switching element (36) can be brought into a third position,
wherein in the third position a rotary movement of the joint head (23) in the receiving area (16) of the joint body (15) is released in the first direction (44) and the second direction (45).

3. Joint (10) according to one of the preceding claims,
wherein at least one blocking element (26) is prestressed by at least one prestressing element (42).

4. Joint (10) according to one of the preceding claims,
wherein
the first blocking element (27) and the second blocking element (28) are each prestressed by a prestressing element (42).

5. Joint (10) according to claim 4,
wherein the switching element (36) has a pressure section (38),
wherein the pressure section (38) is arranged between the first blocking element (27) and the second blocking element (28),
wherein the pressure section (38) is configured in such a way that the pressure section (38) in the first position of the switching element (36) presses laterally against the first blocking element (27), so that the first blocking element (27) is pressed away from the gear wheel section (24) of the joint head (23) against the prestressing,
and wherein the pressure section (38) is configured in such a way that the pressure section (38) in the second position of the switching element (36) presses laterally against the second blocking element (28), so that the second blocking element (28) is pressed away from the gear wheel section (24) of the joint head (23) against the prestressing.

6. Joint (10) according to claim 5,
wherein
the switching element (36) can be brought into a third position and
the pressure section (38) is configured in such a way that the pressure section (38) in the third position of the switching element (36) presses laterally against both blocking elements (26),
so that the two blocking elements (26) can be pressed away from the gear wheel section (24) of the joint head (23) against the respective prestressing.

7. Joint (10) according to one of claims 5 or 6,
wherein
the pressure section (38) has the shape of a segment of a circle, so that the pressure section (38) has two radially outer ends in the circumferential direction, wherein the ends serve for lateral pressing against one blocking element (26) each.

8. Joint (10) for an orthopedic Quengel splint (11), wherein
the joint has a joint head (23) with a gear wheel section (24), a joint body (15) and a single blocking element (26) with a first gear wheel section (30) and a second gear wheel section (31),
wherein the joint body (15) has a receiving area (16), wherein the joint head (23) is rotatably arranged in the receiving area (16), wherein the blocking element (26) is arranged pivotably in the receiving area (16), and wherein the blocking element (26) can be brought into at least a first position and a second position,
wherein in the first position of the blocking element (26) a rotary movement of the joint head (23) in the receiving area (16) is released in the first direction (44) and a rotational movement in the opposite second direction (45) is blocked by an engagement of the first gear section (30) of the blocking element (26) and the gear section (24) of the joint head (23),
and wherein in the second position a rotational movement of the joint head (23) in the receiving area (16) is released in the second direction (45) and a rotational movement in the opposite first direction (44) is blocked by an engagement of the second gear wheel section (31) of the blocking element (26) and the gear wheel section (24) of the joint head (23).

9. Joint (10) according to claim 8,
wherein
the blocking element (26) can be brought into a third position, wherein
in the third position of the blocking element (26) no gear wheel section of the blocking element (26) is in engagement with the gear wheel section (24) of the joint head (23).

10. Joint (10) according to one of claims 8 or 9,
wherein
the blocking element (26) has a switching lever (37).

11. Joint (10) according to one of the preceding claims,
wherein the single blocking element (26) or the switching element (36) can be fixed in a position by at least one pressure element.

12. Joint (10) according to claim 11,
wherein
the pressure element is configured as a resilient pressure piece (46) with a locking ball (47).

13. Joint (10) according to claim 12,
wherein
the joint body (15) has depressions (48) on an inner side (15a),
wherein the resilient pressure piece (46) is arranged in such a way that the locking ball (47), depending on the position of the blocking element (26), slides into in a depression (48) of the joint body (15) to hold the blocking element (26) in position.

14. Orthopedic Quengel splint comprising at least one joint according to one of claims 1 to 13,
wherein the orthopedic Quengel splint comprises two splint elements, wherein a first splint element (12) is solidly connected to the joint body (15), and wherein a second splint element (13) is solidly connected to the joint head (23).

## Revendications

1. Articulation (10) pour attelle orthopédique Quengel (11),
l'articulation (10) comprenant une tête d'articulation (23) dotée d'une section à roue dentée (24), un corps d'articulation (15) et un élément de commutation (36) et un premier élément de blocage (27) et un second élément de blocage (28), le premier élément de blocage (27) et le second élément de blocage (28) présentant respectivement une section à roue dentée (29),
le corps d'articulation (15) présentant une zone de réception (16),
la tête d'articulation (23) étant disposée de manière rotative dans la zone de réception (16),
les éléments de blocage étant disposés de manière pivotante dans la zone de réception (16), et
l'élément de commutation (36) pouvant être amené dans au moins une première position et une deuxième position,
sachant que, dans la première position de l'élément de commutation (36), un mouvement de rotation de la tête d'articulation (23) dans la zone de réception (16) est permis dans le premier sens (44) et qu'un mouvement de rotation dans le second sens opposé (45) est bloqué par un engrènement de la section à roue dentée (29) du premier élément de blocage (27) et de la section à roue dentée (24) de la tête d'articulation (23) et que, dans la deuxième position de l'élément de commutation, un mouvement de rotation de la tête d'articulation (23) dans la zone de réception (16) dans le second sens (45) est permis et qu'un mouvement de rotation dans le premier sens opposé (44) est bloqué par un engrènement de la section à roue dentée (29) du second élément de blocage (28) et de la section à roue dentée (24) de la tête d'articulation (23).

2. Articulation (10) selon la revendication 1, dans laquelle l'élément de commutation (36) peut être amené dans une troisième position,
dans la troisième position, un mouvement de rotation de la tête d'articulation (23) dans la zone de réception (16) du corps d'articulation (15) dans le premier sens (44) et le second sens (45) étant permis.

3. Articulation (10) selon une des revendications précédentes, dans laquelle au moins un élément de blocage (26) est précontraint par au moins un élément de précontrainte (42).

4. Articulation (10) selon une des revendications précédentes, dans laquelle le premier élément de blocage (27) et le second élément de blocage (28) sont précontraints respectivement par un élément de précontrainte (42).

5. Articulation (10) selon la revendication 4, dans laquelle
l'élément de commutation (36) présente une section de compression (38),
la section de compression (38) est disposée entre le premier élément de blocage (27) et le second élément de blocage (28),
la section de compression (38) est réalisée de manière à ce que la section de compression (38), dans la première position de l'élément de commutation (36), appuie latéralement contre le premier élément de blocage (27), de sorte que le premier élément de blocage (27) est repoussé à l'encontre de la précontrainte de la section à roue dentée (24) de la tête d'articulation (23),
et la section de compression (38) est réalisée de manière à ce que la section de compression (38), dans la deuxième position de l'élément de commutation (36), appuie latéralement contre le second élément de blocage (28), de sorte que le second élément de blocage (28) est repoussé à l'encontre de la précontrainte de la section à roue dentée (24) de la tête d'articulation (23).

6. Articulation (10) selon la revendication 5, dans laquelle
l'élément de commutation (36) peut être amené dans une troisième position et la section de compression (38) est réalisée de manière à ce que la section de compression (38) appuie, dans la troisième position de l'élément de commutation (36), latéralement contre les deux éléments de blocage (26),
de sorte que les deux éléments de blocage (26) sont repoussés à l'encontre de la précontrainte de la section à roue dentée (24) de la tête d'articulation (23).

7. Articulation (10) selon une des revendications 5 ou 6, dans laquelle la section de compression (38) présente la forme d'un segment d'arc de cercle, de sorte que la section de compression (38) présente, dans le sens circonférentiel, deux extrémités radialement extérieures, les extrémités servant à la compression latérale respectivement contre un élément de blocage (26).

8. Articulation (10) pour attelle orthopédique Quengel (11),
l'articulation présentant une tête d'articulation (23) dotée d'une section à roue dentée (24), un corps d'articulation (15) et un unique élément de blocage (26) doté d'une première section de roue dentée (30) et d'une seconde section de roue dentée (31),
le corps d'articulation (15) présentant une zone de réception (16), la tête d'articulation (23) étant disposée de manière rotative dans la zone de réception (16), l'élément de blocage (26) étant disposé de manière pivotante dans la zone de réception (16), et l'élément de blocage (26) pouvant être amené dans au moins une première position et une deuxième position,
dans la première position de l'élément de blocage (26), un mouvement de rotation de la tête d'articulation (23) dans la zone de réception (16) étant permis dans le premier sens (44) et un mouvement de rotation dans le second sens opposé (45) étant bloqué par un engrènement de la section à roue dentée (30) de l'élément de blocage (26) et de la section à roue dentée (24) de la tête d'articulation (23), et,
dans la deuxième position, un mouvement de rotation de la tête d'articulation (23) dans la zone de réception (16) dans le second sens (45) étant permis et un mouvement de rotation dans le premier sens opposé (44) étant bloqué par un engrènement de la deuxième section à roue dentée (31) de l'élément de blocage (26) et de la section à roue dentée (24) de la tête d'articulation (23).

9. Articulation (10) selon la revendication 8, dans laquelle
l'élément de blocage (26) peut être amené dans une troisième position, sachant que, dans la troisième position de l'élément de blocage (26), aucune section à roue dentée de l'élément de blocage (26) n'est en prise avec la section à roue dentée (24) de la tête d'articulation (23).

10. Articulation (10) selon la revendication 8 ou 9, dans laquelle
l'élément de blocage (26) présente un levier de commutation (37).

11. Articulation (10) selon l'une des revendications précédentes, dans laquelle
l'élément de blocage unique (26) ou l'élément de commutation (36) peut être fixé dans une position par au moins un élément de compression.

12. Articulation (10) selon la revendication 11, dans laquelle
l'élément de compression se présente sous forme d'une pièce de compression à ressort (46) dotée d'une bille d'arrêt (47).

13. Articulation (10) selon la revendication 12, dans laquelle
le corps d'articulation (15) présente des creux (48) sur une face intérieure (15a),
la pièce de compression à ressort (46) est disposée de manière à ce que la bille d'arrêt (47), en fonction de la position de l'élément de blocage (26), glisse dans un creux (48) du corps d'articulation (15) afin de maintenir l'élément de blocage (26) dans la position.

14. Attelle orthopédique Quengel, comprenant au moins une articulation selon l'une quelconque des revendications 1 à 13,
l'attelle orthopédique Quengel présentant deux éléments d'attelle, un premier élément d'attelle (12) étant connecté rigidement au corps d'articulation (15), et un second élément d'attelle (13) étant connecté rigidement à la tête d'articulation (23).
